# FASCICULE DE BREVET EUROPEEN

(11) **EP 4 090 220 B1**
(45) Date de publication et mention de la délivrance du brevet: **30.07.2025**
(21) Numéro de dépôt: 21719946.2
(22) Date de dépôt: 12.01.2021
(51) Int. Cl.: A61B 1/00, F16M 11/04

(54) **SYSTEME D'ASSEMBLAGE D'UN APPAREIL NOTAMMENT MEDICAL AVEC UN SUPPORT**
SYSTEM ZUM ZUSAMMENBAU EINES GERÄTES, INSBESONDERE EINES MEDIZINISCHEN GERÄTES, MIT EINEM TRÄGER
SYSTEM FOR ASSEMBLING OF A DEVICE, IN PARTICULAR OF A MEDICAL DEVICE, WITH A SUPPORT

(30) Priorité: 17.01.2020 FR 2000448
(43) Date de publication de la demande: 23.11.2022
(73) Titulaire: Axess Vision Technology, 37300 Joue les Tours (FR)
(72) Inventeur: HALLAUER, Emmanuel, 37190 SACHE (FR); LE ROUX, Philippe, 37100 TOURS (FR)
(74) Mandataire: Cabinet Beau de Loménie
(86) Numéro de dépôt international: PCT/FR2021/050051
(87) Numéro de publication internationale: WO 2021/144530

(56) Documents cités:
- EP-A1- 2 803 585
- EP-A1- 3 029 543
- WO-A2-2018/204937
- US-A1- 2008 315 049
- US-A1- 2014 360 893
- US-A1- 2015 136 933
- US-A1- 2019 154 190

## Description

### Domaine Technique

La présente invention concerne le domaine technique des systèmes de fixation permettant de rendre solidaire temporairement d'un support, un appareil électronique au sens général.

La présente invention trouve des applications dans de nombreux domaines industriels mais elle vise plus particulièrement le domaine médical où un appareil électronique médical est à accrocher temporairement sur un support, tel un pied à perfusion ou une tige à soluté par exemple.

L'objet de l'invention trouve une application particulièrement avantageuse dans le domaine où l'appareil médical électronique est un écran de visualisation utilisé avec un dispositif médical, tel qu'un endoscope, cet écran étant destiné à être suspendu temporairement lors de l'utilisation de l'endoscope.

Un endoscope médical au sens général permet d'accéder à l'intérieur d'un organe creux, d'une cavité ou d'un conduit naturel ou artificiel du corps humain en vue d'effectuer diverses opérations à des fins thérapeutiques, chirurgicales ou de diagnostic.

### Technique antérieure

D'une manière générale, un endoscope comporte une poignée de commande se présentant sous la forme d'un boîtier adapté pour être tenu par un utilisateur de l'endoscope et auquel est fixée une structure tubulaire conçue pour être insérée dans une cavité corporelle d'un patient à examiner. Cette structure tubulaire d'insertion comporte une tête distale équipée d'un système de vision permettant d'éclairer et d'examiner l'organe, la cavité ou le conduit du corps humain. En amont de la tête distale, la structure tubulaire d'insertion comporte une partie de béquillage commandée par un mécanisme de la poignée de commande pour orienter la tête distale à l'intérieur de la voie d'insertion. Afin de visualiser l'intérieur de l'organe creux, la cavité ou le conduit naturel ou artificiel du corps humain dans lequel l'endoscope est inséré, l'endoscope est relié à un écran de visualisation sur lequel sont affichées les images provenant du système de vision.

Des écrans de visualisation fixes sont très répandus : il s'agit d'écrans fixés au mur de la salle d'opération par exemple. La stabilité de ces écrans est alors garantie. Cependant, afin de réaliser une endoscopie, il est alors indispensable d'être dans une salle équipée d'un écran de visualisation fixe. Dans un hôpital ou une clinique, cela impose soit d'équiper toutes les salles en écrans de visualisation, soit de limiter la réalisation d'endoscopie à certaines salles. C'est pourquoi, il est avantageux de pouvoir disposer d'un écran de visualisation mobile.

Des écrans de visualisation mobiles sont également connus. Il s'agit notamment d'écrans sur pied, pouvant être posés sur un plan de pose présent dans la salle d'opération. Selon les dimensions et le poids de ces écrans, leur déplacement n'est pas toujours aisé.

Il existe en particulier l'écran de visualisation mobile commercialisé par la société AMBU^{®} sous la dénomination AVIEW^{™}, qui est mobile et de faible encombrement. Cet écran de visualisation présente l'avantage non seulement de pouvoir être posé sur un plan de pose, mais également de pouvoir être fixé sur une tige à soluté. Pour cela, l'écran de visualisation comporte sur sa face arrière un système de fixation à vis comportant un arceau dont l'une des branches est munie d'une vis dont le serrage sur la tige à soluté permet de suspendre l'écran tout en pouvant régler sa position par rapport à la tige. Pour rendre l'écran facilement détachable par rapport au système de fixation à vis, le dos de l'écran est pourvu d'une glissière destinée à coopérer avec une glissière complémentaire aménagée sur le système de fixation à vis. Il s'avère en pratique que le montage et le démontage de l'écran sont des opérations relativement délicates à mener à bien car elles nécessitent d'aligner parfaitement la glissière du dos de l'écran avec la glissière du support. En dehors de ces difficultés de manipulation, les opérations de montage et de démontage s'accompagnent d'un risque important de chute de l'écran et par suite de détérioration de l'écran.

Il est également connu par la demande de brevet US 2019/154190, un système pour monter sur un véhicule, un appareil électronique. Ce système de fixation comporte une partie de fixation solidaire de l'appareil électronique et comportant des saillies et des rainures disposées de manière alternée. Ce système comporte également un capuchon de verrouillage pourvu d'une saillie et d'un capuchon rotatif présentant une rainure et monté tournant par rapport au capuchon de verrouillage. Le support est fixé à la partie de fixation en insérant la saillie du capuchon de verrouillage dans une rainure de la partie de fixation et en faisant tourner relativement la partie de fixation par rapport au capuchon de verrouillage. Lors de cette rotation, une languette d'un organe de verrouillage coopère avec la rainure du capuchon rotatif, empêchant le retrait du capuchon de verrouillage par rapport à la partie de fixation. Un tel système de fixation présente une conception relativement complexe et encombrante avec un organe de verrouillage faisant saillie de l'appareil électronique pour être actionné.

La demande de brevet US 2014/360893 concerne un dispositif de montage d'une coque de protection d'un appareil électronique portatif sur un support. Cette coque de protection comporte dans sa paroi arrière, un logement délimité par des dents espacées et dans lequel est inséré un élément de verrouillage pourvu de pattes qui après rotation sont bloquées derrière les dents de la coque de protection. Un tel dispositif présente également l'inconvénient que l'élément de déverrouillage dépasse de l'appareil électronique pour pouvoir être actionné.

La demande de brevet US 2015/136933 décrit un système de connexion pour monter de manière amovible un dispositif électronique portatif sur une surface plane. Ce système comporte un connecteur mâle pourvu de dents destiné à s'engager dans un connecteur femelle présentant des logements de forme complémentaires aux dents. Une rotation relative du connecteur mâle par rapport au connecteur femelle permet une solidarisation entre eux. Cependant, un tel système de connexion n'assure pas une immobilisation sûre et une immobilisation en rotation du dispositif électronique portatif par rapport à la surface plane.

### Exposé de l'invention

La présente invention vise donc à remédier aux inconvénients de l'état de la technique en proposant un nouveau système d'assemblage amovible conçu pour permettre un montage et un démontage facile d'un appareil sur un support, ces opérations de montage et de démontage pouvant être réalisées en parfaite sécurité pour l'appareil.

La présente invention vise également à proposer un nouveau système d'assemblage amovible conçu pour permettre avec une seule main, d'assurer un montage et un démontage d'un appareil sur un support.

Pour atteindre un tel objectif, le système selon l'invention vise à assurer l'assemblage amovible d'un appareil avec un support, à l'aide d'une partie mâle équipant le support et coopérant avec une partie femelle équipant l'appareil :
- la partie mâle comportant un bras présentant une extrémité libre pourvue d'une série de dents d'appui s'étendant radialement dans un plan radial en étant distribués selon une répartition angulaire ;
- la partie femelle comportant au moins une plaque de blocage délimitée par une face avant et une face arrière et dans lequel est aménagée une ouverture de section adaptée pour autoriser dans une position d'insertion, l'engagement à partir de la face avant, de l'extrémité libre de la partie mâle au-delà de la face arrière, l'ouverture aménagée dans la plaque de blocage délimitant des dents de retenue s'étendant radialement et qui, après une rotation relative entre la partie femelle et la partie mâle, sont aptes dans une position de blocage, à venir en vis-à-vis d'une dent d'appui pour interdire le dégagement entre les parties mâle et femelle, de la face arrière en direction de la face avant ;
- la partie femelle et la partie mâle comportant un dispositif de verrouillage angulaire apte à verrouiller angulairement les parties femelle et mâle lorsqu'elles occupent la position de blocage, le dispositif de verrouillage angulaire comportant un loquet monté mobile radialement sur la face arrière de la plaque de blocage et pourvu d'un organe de déplacement en translation accessible à partir de la face avant de la plaque de blocage, le loquet étant destiné à s'engager automatiquement dans une gorge d'arrêt du bord externe d'une dent d'appui ;
- la partie femelle comportant un fond de butée pour la partie mâle limitant l'engagement de la partie mâle au-delà de la face arrière de la plaque de blocage et présentant une surface de guidage en rotation entre la partie mâle et la partie femelle afin de passer de la position d'insertion à la position de blocage et inversement.

De plus, le système selon l'invention peut comporter les caractéristiques additionnelles suivantes :
- le fond de butée comporte en tant que surface de guidage en rotation, un plot circulaire de forme conique ;
- la partie mâle comporte un nombre de dents d'appui égal au nombre de dents de retenue de la partie femelle ;
- les dents d'appui de la partie mâle et les dents de retenue de la partie femelle sont réparties régulièrement angulairement ;
- deux dents de retenue voisines de la partie femelle délimitent entre elles une encoche de passage pour une dent d'appui de la partie mâle tandis que deux dents d'appui voisines de la partie mâle délimitent entre elles un trou de passage pour une dent de retenue de la partie femelle ;
- la largeur angulaire d'une encoche de passage prise entre deux dents de retenue voisines est égale à une valeur comprise entre 105 à 200% de la largeur angulaire prise pour une dent d'appui ;
- le bord externe des dents d'appui comporte à partir de chacune de ses extrémités, une rampe d'appui pour le loquet débouchant dans une gorge d'arrêt du loquet ;
- le loquet est sollicité élastiquement en extension par un organe ressort dans sa position d'extension maximale de manière à être comprimée progressivement lorsqu'il est en appui sur la rampe du bord externe jusqu'à venir s'engager automatiquement dans la gorge d'arrêt ;
- le loquet est destiné à s'engager automatiquement dans une gorge d'arrêt du bord externe d'une dent d'appui sous l'effet de son propre poids ;
- le loquet est monté pour s'étendre verticalement à l'aplomb du milieu d'une dent de retenue aménagée dans la plaque de blocage.

Un autre objet de l'invention est de proposer un matériel comportant un appareil assemblé avec un support à l'aide d'un système d'assemblage amovible conforme à l'invention.

Selon une variante avantageuse de réalisation, l'appareil comporte un boîtier avec une face arrière pourvue de la partie femelle du système d'assemblage dont la partie mâle équipe le support. Avantageusement, la face arrière du boîtier de l'appareil est pourvue de l'organe de déplacement en translation du loquet de verrouillage, positionné à proximité du bord supérieur du boîtier.

Selon une application préférée, l'appareil est un écran de visualisation d'un endoscope médical.

Diverses autres caractéristiques ressortent de la description faite ci-dessous en référence aux dessins annexés qui montrent, à titre d'exemples non limitatifs, des formes de réalisation de l'objet de l'invention.

### Brève description des dessins

[Fig. 1]La Figure 1 est une vue en perspective de trois quart arrière d'un appareil médical réalisé sous la forme d'un écran de visualisation équipé de la partie femelle du système d'assemblage amovible conforme à l'invention.
[Fig. 2] La Figure 2 est une vue du dos de l'appareil médical illustré à la Figure 1.
[Fig. 3]La Figure 3 est une vue de l'arrière du dos de l'appareil médical illustré à la Figure 2.
[Fig. 4]La Figure 4 est une vue en perspective de la partie mâle du système d'assemblage amovible conforme à l'invention.
[Fig. 5]La Figure 5 est une vue en perspective montrant l'engagement de la partie mâle dans la partie femelle du système d'assemblage amovible conforme à l'invention.
[Fig. 6]La Figure 6 est une vue en perspective montrant après l'engagement de la partie mâle dans la partie femelle du système d'assemblage amovible conforme à l'invention, la position de la partie femelle par rapport à la partie mâle après une rotation relative.
[Fig. 7]La Figure 7 est une vue en perspective montrant le verrouillage de la partie mâle dans la partie femelle du système d'assemblage amovible conforme à l'invention.
[Fig. 8]La Figure 8 est une vue en perspective montrant la première phase de déverrouillage de la partie mâle par rapport à la partie femelle du système d'assemblage amovible conforme à l'invention.

### Description des modes de réalisation

L'objet de l'invention concerne un système d'assemblage amovible 1 permettant d'assurer un assemblage démontable d'un appareil 2 sur un support 3 de tous types connus en soi. Selon une application préférée, le support 3 relève du domaine médical. A titre d'exemple, le support 3 peut être un accessoire médical appelé communément pied à perfusion ou tige à soluté. Bien entendu, le support 3 peut être réalisé de manière différente comme sous la forme d'un meuble ou d'un chariot. Le support 3 est schématisé sur les dessins sous la forme d'un corps cylindrique uniquement à titre d'illustration.

Le système d'assemblage amovible 1 conforme à l'invention permet d'accrocher de manière temporaire sur le support 3, un appareil 2 de tous types. Selon un exemple d'application préférée, le système d'assemblage amovible 1 permet l'accrochage, d'un appareil électronique médical comme un moniteur médical ou avantageusement, un écran de visualisation 2 destiné à être relié à un endoscope médical de tous types connus en soi. Cet écran de visualisation 2 peut ainsi être fixé de manière temporaire sur le support 3 (pied à perfusion ou tige à soluté par exemple) notamment lors de l'utilisation de l'endoscope médical auquel est relié l'écran de visualisation 2.

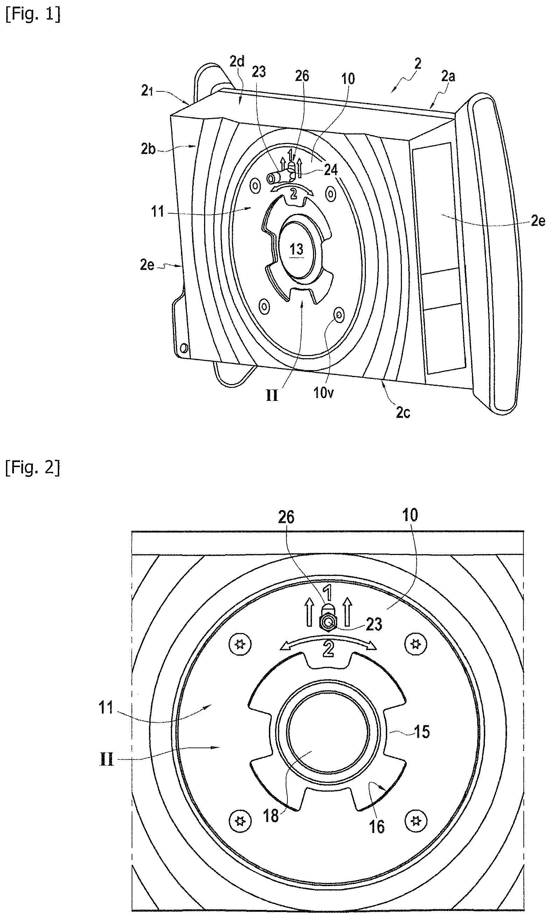

Tel que cela ressort plus précisément des Figures 1 à 8, le système d'assemblage amovible 1 comporte deux parties indépendantes à savoir une partie mâle I et une partie femelle II destinées à coopérer ensemble pour permettre la fixation de l'appareil 2 sur le support 3. Dans l'exemple de réalisation illustré sur les dessins, l'appareil 2 est pourvu de la partie femelle II tandis que le support 3 est muni de la partie mâle I. Bien entendu, il peut être prévu une autre variante de réalisation pour laquelle l'appareil 2 est pourvu de la partie mâle I tandis que le support 3 est muni de la partie femelle II.

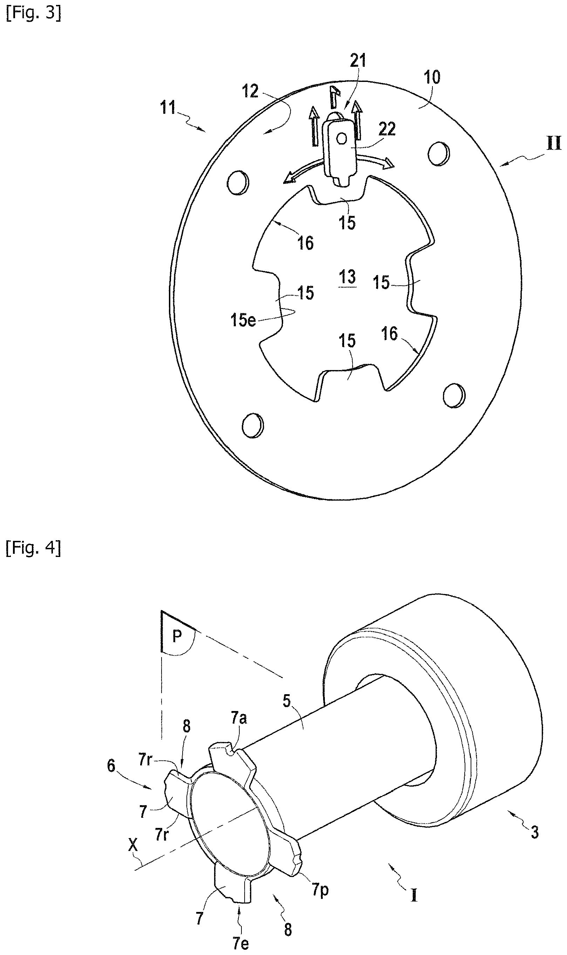

Tel que cela ressort plus précisément de la Figure 4, la partie mâle I comporte un bras 5 présentant une extrémité libre 6 et s'étendant longitudinalement selon un axe longitudinal qui sera appelé axe d'insertion X dans la suite de la description. L'extrémité libre 6 du bras 5 est pourvue d'une série de dents d'appui 7 s'étendant perpendiculairement par rapport à l'axe d'insertion X. Les dents d'appui 7 s'étendent radialement dans un plan radial P perpendiculaire à l'axe d'insertion X. Dans l'exemple illustré sur les dessins, le bras 5 possède une forme tubulaire de section circulaire mais il est clair que le bras 5 peut présenter une forme différente.

Les dents d'appui 7 sont distribuées à la périphérie du bras 5 selon une répartition angulaire déterminée, de préférence régulière. Selon l'exemple illustré, le bras 5 est équipé de quatre dents d'appui 7 décalés angulairement deux à deux de 90°. Il est clair que le nombre de dents d'appui 7 peut être différent de quatre et peut être compris par exemple entre 2 et 10. Deux dents d'appui 7 voisines délimitent entre elles un trou de passage 8. Dans l'exemple illustré, l'extrémité libre du bras 6 comporte quatre dents d'appui 7 délimitant quatre trous de passage 8.

La largeur angulaire des dents d'appui 7 peut prendre différentes valeurs adaptées afin que les dents d'appui 7 assurent leur fonction de blocage comme cela sera mieux compris dans la suite de la description. De préférence mais non exclusivement, la largeur angulaire est identique pour toutes les dents d'appui 7. Par exemple, la largeur angulaire d'une dent d'appui 7 est égale à une valeur comprise entre 0.5 à 0.9 fois la largeur angulaire d'un trou de passage 8.

Chaque dent d'appui 7 est délimitée par un bord externe 7e. Chaque dent d'appui 7 peut présenter une forme quelconque. Dans l'exemple illustré, chaque dent d'appui 7 possède une forme sensiblement rectangulaire. Selon cet exemple, le bord externe 7e de chaque dent d'appui 7 comporte de part et d'autre, deux parties radiales 7r reliées entre elles par une partie périphérique 7p.

Selon une variante avantageuse de réalisation, la partie périphérique 7p du bord externe 7e comporte à partir de chaque partie radiale 7r, une rampe d'appui dont la hauteur par rapport au bras 5, augmente en direction de la partie médiane de la partie périphérique 7p. Les deux rampes d'appui se rejoignent au milieu de la partie périphérique 7p du bord externe 7e, au niveau d'une gorge d'arrêt 7a dont la fonction apparaîtra plus précisément dans la suite de la description.

La partie femelle II comporte au moins une plaque de blocage se présentant par exemple sous la forme d'un disque de blocage 10 délimité par une face avant 11 et une face arrière 12, et dans lequel est aménagée une ouverture 13 de section adaptée pour autoriser dans une position d'insertion, l'engagement à partir de la face avant 11, de l'extrémité libre 6 de la partie mâle II au-delà de la face arrière 12. Les faces avant 11 et arrière 12 sont prises en considération de la position de la partie mâle I par rapport à la partie femelle II, avant l'insertion de la partie mâle I dans la partie femelle II.

En d'autres termes, la face avant 11 correspond à la face dirigée vers l'extérieur telle que la face externe de la paroi arrière de l'appareil 2 dans l'exemple illustré alors que la face arrière 12 correspond à la face interne de la paroi arrière de l'appareil 2. L'insertion de la partie mâle I dans la partie femelle II est réalisée par un déplacement relatif selon l'axe d'insertion X, entre la partie mâle I et la partie femelle II. Pour obtenir cette insertion, la partie mâle est déplacée par rapport à la partie femelle, la partie femelle est déplacée par rapport à la partie mâle ou la partie mâle et la partie femelle sont déplacées simultanément.

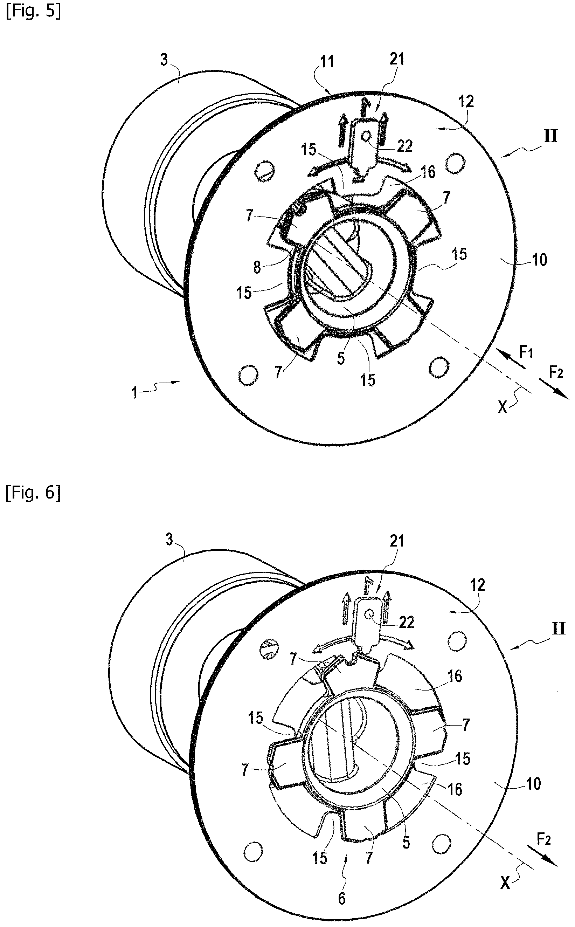

A titre d'exemple dans la suite de la description, le sens d'engagement est noté par la flèche F1 et correspond au déplacement de la partie femelle II pour obtenir l'insertion de la partie mâle I dans la partie femelle II, à partir de la face avant 11 de la partie femelle (Figure 5). A contrario, le sens de dégagement est noté par la flèche F2 et correspond au déplacement de la partie femelle II pour obtenir le retrait par rapport à la partie mâle, de la partie femelle II selon un sens contraire au sens d'engagement F1.

Pour permettre l'insertion de la partie mâle dans la partie femelle, il doit être compris que la section de passage de l'ouverture 13 du disque de blocage 10 est supérieure à la section de l'extrémité libre 6 de la partie mâle II, prise dans le plan radial et formée par la section du bras 5 et par la section ou la surface des dents d'appui 7. En d'autres termes, la section de l'extrémité libre 6 de la partie mâle II prise dans le plan radial comprenant le bras 5 et les dents d'appui 7 est incluse strictement dans la section de passage de l'ouverture 13.

L'ouverture 13 est aménagée dans le disque 10 pour délimiter des dents de retenue 15 s'étendant radialement (Figure 3). Les dents de retenue 15 sont distribuées selon une circonférence du disque 10 avec une répartition angulaire déterminée, de préférence régulière. Selon l'exemple illustré, le disque 10 est équipé de quatre dents de retenue 15 décalés angulairement deux à deux de 90°. Il est clair que le nombre de dents de retenue 15 peut être différent de quatre et peut être compris par exemple entre 2 et 10. Selon l'exemple de réalisation, la partie femelle II comporte un nombre de dents de retenue 15 égal au nombre de dents d'appui 7 de la partie mâle I. Bien entendu, le nombre de dents de retenue 15 peut être différent du nombre de dents d'appui 7 de la partie mâle I.

Chaque dent de retenue 15 est délimitée par un bord externe 15e. Chaque dent de retenue 15 peut présenter une forme quelconque. Dans l'exemple illustré, chaque dent de retenue 15 possède une forme sensiblement rectangulaire. Deux dents de retenue 15 voisines délimitent entre elles une encoche 16 de passage pour une dent d'appui 7 de la partie mâle I. Selon une caractéristique avantageuse de réalisation, la largeur angulaire d'une encoche 16 prise entre deux dents de retenue 15 voisines est égale à une valeur comprise entre 105 à 200 % de la largeur angulaire d'une dent d'appui 7.

Ainsi, lors de l'engagement de la partie mâle I dans la partie femelle II selon l'axe d'insertion X, l'extrémité distale 6 est apte à traverser l'ouverture 13. Ainsi, les dents d'appui 7 traversent l'ouverture 13 par les encoches 16 tandis que les trous 8 autorisent le passage des dents de retenue 15. Les dents d'appui 7 peuvent ainsi s'étendre au-delà ou derrière la face arrière 12 du disque de blocage 10 de manière à permettre une rotation relative entre la partie mâle I et la partie femelle II autour de l'axe d'insertion X. Bien entendu, la partie femelle II délimite à partir de la face arrière 12 du disque de blocage 10, un dégagement autorisant le positionnement et la rotation de l'extrémité libre 6 du bras 5 pourvue des dents d'appui 7.

Les dents de retenue 15 sont aménagés de manière qu'après une rotation relative entre la partie femelle II et la partie mâle I, au moins une et de préférence l'ensemble des dents d'appui 7 sont aptes à être positionnés au moins en partie en vis-à-vis des dents de retenue 15 pour interdire le dégagement entre les parties mâle I et femelle II, selon le sens de dégagement F2 contraire au sens d'insertion F1 (Figure 6). Selon l'exemple préféré de réalisation illustré sur les dessins, chaque dent de retenue 15 est bloquée par une dent d'appui 7 pour un déplacement de la partie femelle II dans le sens de dégagement F2. Lorsque la partie mâle I et la partie femelle II occupent cette position, le système d'assemblage amovible 1 est dans une position de blocage en translation.

Selon l'invention la partie femelle II comporte un fond de butée 18 pour la partie mâle I limitant l'engagement de la partie mâle au-delà de la face arrière 12 du disque de blocage 10. Ce fond de butée 18 est positionné pour permettre le positionnement des dents d'appui 7 derrière les dents de retenue 15 en restant en contact ou en appui avec ces dernières. Ce fond de butée 18 peut exercer un effort d'appui dans le sens contraire au sens d'insertion pour assurer un appui des dents d'appui 7 contre les dents de retenu 15. Ce fond de butée 18 présente une capacité de compression de par son matériau constitutif ou se trouve sollicité par un organe ressort par exemple.

Selon l'invention, le fond de butée 18 présente une surface de guidage en rotation entre la partie mâle I et la partie femelle II afin de permettre le passage de la position d'insertion à la position de blocage et inversement. Par exemple, le fond de butée 18 est réalisé par un plot circulaire de forme conique sur laquelle prend appui l'extrémité distale du bras tubulaire 5, favorisant le guidage en rotation et le centrage en approche des deux pièces, à savoir la partie mâle et la partie femelle.

Selon une autre caractéristique de l'invention, la partie femelle II et la partie mâle I comportent un dispositif de verrouillage angulaire 21 apte à verrouiller angulairement les parties femelle II et mâle I lorsqu'elles occupent la position de blocage. Selon l'exemple de réalisation illustré sur les Figures, le dispositif de verrouillage angulaire 21 comporte un loquet 22 monté mobile radialement sur la face arrière 12 du disque de blocage 10. Avantageusement, ce loquet 22 est monté pour s'étendre verticalement, à l'aplomb du milieu d'une dent de retenue 15 et de préférence, de la dent de retenue 15 située au niveau le plus élevé verticalement. Tel que cela ressort des figures, le loquet 22 s'étend radialement en retrait du bord intérieur délimité par l'ouverture 13 et en particulier par une dent de retenue 15 et également en retrait du bord extérieur du disque de blocage 10 de sorte que le loquet 22 est totalement invisible depuis la face avant 11 du disque de blocage 10.

Ce loquet 22 est pourvu d'un organe 23 permettant de le déplacer en translation et positionné pour être accessible à partir de la face avant 11 du disque de blocage 10. A cet effet, le loquet 22 est pourvu d'une tige 24 reliée à l'organe de déplacement 23 s'étendant en saillie par rapport à la face avant 11. La tige 24 traverse une lumière oblongue 26 pratiquée dans le disque de blocage 10 pour autoriser le déplacement radial du loquet 22. Le loquet 22 est destiné à coopérer par sa partie terminale, avec une dent d'appui 7 pour assurer le blocage en rotation de la partie mâle I par rapport à la partie femelle II. Comme cela sera expliqué dans la suite de la description, l'organe de déplacement 23 est destiné à être déplacé pour déverrouiller le blocage en rotation.

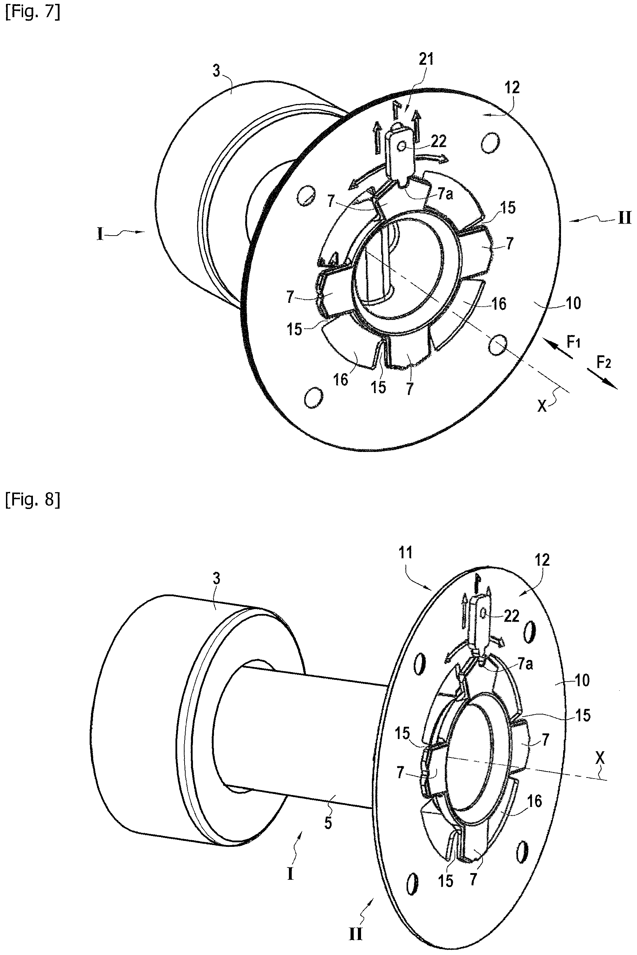

Selon l'invention le loquet 22 est destiné à s'engager dans la gorge d'arrêt 7a de la dent d'appui situé en correspondance. Lors de la rotation relative entre les parties mâle et femelle, le loquet 22 est en contact avec le bord externe 7e d'une dent d'appui 7 et vient s'engager dans la gorge d'arrêt 7a pour bloquer la rotation (Figure 7). Le loquet 22 s'engage automatiquement dans la gorge d'arrêt 7a sous l'effet de son poids ou le loquet 22 est sollicité élastiquement par un organe ressort dans sa position d'extension maximale de manière à être comprimé progressivement lorsqu'il est en appui sur la rampe du bord externe 7e jusqu'à venir s'engager automatiquement dans la gorge d'arrêt 7a. Le verrouillage angulaire est réalisé automatiquement lors de la rotation relative entre les parties mâle et femelle.

Bien entendu, le déblocage en rotation de la partie mâle I par rapport à la partie femelle II consiste à soulever l'organe d'actionnement 23 pour dégager le loquet 22 de la gorge d'arrêt 7a (Figure 8). Il devient alors possible de tourner relativement entre elles les parties femelle et mâle.

Dans l'exemple de réalisation illustré, le loquet 22 comporte une partie terminale saillante destinée à s'engager dans une gorge d'arrêt 7a. Bien entendu, le loquet 22 peut présenter une partie terminale réalisée avec une forme différente comme par exemple, sous la forme d'une griffe venant s'engager de part et d'autre d'un bourrelet porté par la dent d'appui 7. Dans un exemple qui n'est pas couvert par les revendications, le dispositif de verrouillage angulaire 21 peut être différent d'un loquet agissant radialement et verticalement sur une dent d'appui 7. Ainsi, le dispositif de verrouillage angulaire 21 peut être réalisé par exemple, par un loquet agissant axialement pour s'engager dans un trou aménagé dans la dent d'appui 7 placée en correspondance.

La mise en œuvre du système d'assemblage amovible 1 conforme à l'invention découle directement de la description qui précède. L'assemblage de la partie mâle I avec la partie femelle II consiste à positionner l'extrémité terminale 6 de la partie mâle devant la face avant 11 de la partie femelle, en alignant les dents d'appui 7 de la partie mâle I avec les encoches 16 de la partie femelle II (Figure 5). Le déplacement relatif selon le sens d'engagement F1 suivant l'axe d'insertion X, entre la partie mâle I et la partie femelle II conduit la partie mâle I et la partie femelle II à occuper une position d'insertion dans laquelle les dents d'appui 7 s'étendent au-delà ou derrière la face arrière 12 du disque de blocage 10 (Figure 6). Avantageusement, la position finale d'insertion selon l'axe d'insertion X est donnée par la mise en butée de la partie terminale 6 de la partie mâle I avec le fond de butée 18 de la partie femelle II. La partie mâle I et la partie femelle II sont donc bloquées selon le sens d'engagement F1.

Dans cette position finale d'insertion, une rotation relative entre la partie mâle I et la partie femelle II conduit le loquet 22 à venir en contact avec le bord externe de la dent d'appui 7 en correspondance. Au cours de cette rotation, le loquet 22 est soulevé progressivement par la rampe aménagée sur le bord externe 7e et vient s'engager automatiquement dans la gorge 7a. Dans cette position, la partie mâle I et la partie femelle II sont donc bloquées en rotation relative. Dans cette position également, les dents d'appui 7 sont situées en regard des dents de retenue 15 de sorte que la partie mâle I et la partie femelle II sont bloquées selon le sens de dégagement F2. Dans la position illustrée à la Figure 7, la partie mâle I et la partie femelle II sont assemblées ensemble par une liaison complète par le système conforme à l'invention.

La séparation entre la partie mâle I et la partie femelle II est réalisée en réalisant des opérations inverses de celles décrites précédemment. Le loquet 22 est soulevé à l'aide de l'organe de déplacement 23 accessible à partir de la face avant 11, pour le dégager de la dent d'appui 7 (Figure 8) permettant une rotation relative entre la partie mâle I et la partie femelle II jusqu'à ce que les dents d'appui 7 se trouvent positionnées en vis-à-vis des encoches 16. Le déplacement relatif selon le sens de dégagement F2 suivant l'axe d'insertion X, entre la partie mâle I et la partie femelle II conduit la partie mâle I et la partie femelle II à occuper une position de dégagement dans laquelle les dents d'appui 7 s'étendent au-delà ou devant la face avant 11 du disque de blocage.

Le système d'assemblage amovible 1 conforme à l'invention permet d'assurer un assemblage démontable d'un appareil 2 sur un support 3 de tous types connus en soi. Selon une application préférée, le support 3 relève du domaine médical tandis que l'appareil 2 est un écran de visualisation destiné à être relié à un endoscope médical de tous types connus en soi. Selon l'exemple illustré à la Figure 1, l'écran de visualisation 2 est équipé de la partie femelle II. A cet effet, l'écran de visualisation 2 comporte un boîtier 2₁ présentant une face avant 2a et une face arrière 2b s'étendant sensiblement en vis-à-vis de la face avant 2a. Le boîtier comprend un bord inférieur 2c et à l'opposé un bord supérieur 2d. Ce bord inférieur 2c et le bord supérieur 2d sont reliés entre eux par deux bords latéraux 2e. Une partie de la face arrière 2b est pourvue de la plaque de blocage 10 avec sa face avant tournée vers l'extérieur du boîtier tandis que le fond de butée 18 est montée à l'intérieur du boîtier. La plaque de blocage 10 est par exemple fixée par des vis 10v sur la face arrière 2b du boîtier. Avantageusement, la plaque de blocage 10 est montée de manière que l'organe de déplacement 23 du loquet de verrouillage 22 se trouve positionné à proximité de préférence du bord supérieur 2d du boîtier. Un tel positionnement permet de déverrouiller le loquet en le soulevant et de prendre simultanément l'écran avec une seule main.

Il ressort de la description qui précède que l'objet de l'invention concerne également un matériel comportant un appareil 2 assemblé à un support 3 au sens général à l'aide d'un système d'assemblage amovible 1 conforme à l'invention. L'appareil 2 est équipé par exemple de la partie femelle II tandis que le support 4 est pourvu de la partie mâle I. L'appareil 2 peut être monté et démonté facilement par rapport au support 3.

L'invention n'est pas limitée aux exemples décrits et représentés, l'invention n'est limitée que par l'ensemble des revendications annexées.

## Revendications

1. Système d'assemblage amovible (1) d'un appareil (2) avec un support (3), à l'aide d'une partie mâle (I) équipant le support (3) et coopérant avec une partie femelle (II) équipant l'appareil (2) ;
- la partie mâle (I) comportant un bras (5) présentant une extrémité libre (6) pourvue d'une série de dents d'appui (7) s'étendant radialement dans un plan radial en étant distribués selon une répartition angulaire ;
- la partie femelle (II) comportant au moins une plaque de blocage (10) délimitée par une face avant (11) et une face arrière (12) et dans lequel est aménagée une ouverture (13) de section adaptée pour autoriser dans une position d'insertion, l'engagement à partir de la face avant, de l'extrémité libre (6) de la partie mâle au-delà de la face arrière, l'ouverture (13) aménagée dans la plaque de blocage (10) délimitant des dents de retenue (15) s'étendant radialement et qui, après une rotation relative entre la partie femelle (II) et la partie mâle (I), sont aptes dans une position de blocage, à venir en vis-à-vis d'une dent d'appui (7) pour interdire le dégagement entre les parties mâle et femelle, de la face arrière en direction de la face avant ;
- la partie femelle (II) et la partie mâle (I) comportant un dispositif de verrouillage angulaire (21) apte à verrouiller angulairement les parties femelle (II) et mâle (I) lorsqu'elles occupent la position de blocage, le dispositif de verrouillage angulaire (21) comportant un loquet (22) monté mobile radialement sur la face arrière (12) de la plaque de blocage (10) et pourvu d'un organe (23) de déplacement en translation accessible à partir de la face avant (11) de la plaque de blocage, le loquet (22) étant destiné à s'engager automatiquement dans une gorge d'arrêt du bord externe (7e) d'une dent d'appui (7) ;
- la partie femelle (II) comportant un fond de butée (18) pour la partie mâle (I) limitant l'engagement de la partie mâle (I) au-delà de la face arrière (12) de la plaque de blocage (10) et présentant une surface de guidage en rotation entre la partie mâle (I) et la partie femelle (II) afin de passer de la position d'insertion à la position de blocage et inversement.

2. Système d'assemblage selon la revendication précédente, selon lequel le fond de butée (18) comporte en tant que surface de guidage en rotation, un plot circulaire de forme conique.

3. Système d'assemblage selon l'une des revendications précédentes, selon lequel la partie mâle (I) comporte un nombre de dents d'appui (7) égal au nombre de dents de retenue (15) de la partie femelle.

4. Système d'assemblage selon l'une des revendications précédentes, selon lequel les dents d'appui (7) de la partie mâle (I) et les dents de retenue (15) de la partie femelle (II) sont réparties régulièrement angulairement.

5. Système d'assemblage selon l'une des revendications précédentes, selon lequel deux dents de retenue (15) voisines de la partie femelle (II) délimitent entre elles une encoche (16) de passage pour une dent d'appui (7) de la partie mâle (I) tandis que deux dents d'appui (7) voisines de la partie mâle (I) délimitent entre elles un trou (8) de passage pour une dent de retenue (15) de la partie femelle (II).

6. Système d'assemblage selon la revendication précédente, selon lequel la largeur angulaire d'une encoche (16) de passage prise entre deux dents de retenue (15) voisines est égale à une valeur comprise entre 105 à 200% de la largeur angulaire prise pour une dent d'appui (7).

7. Système d'assemblage selon la revendication précédente, selon lequel le bord externe (7e) des dents d'appui (7) comporte à partir de chacune de ses extrémités, une rampe d'appui pour le loquet (22) débouchant dans une gorge d'arrêt (7a).

8. Système d'assemblage selon la revendication 1, selon lequel le loquet (22) est sollicité élastiquement en extension par un organe ressort dans sa position d'extension maximale de manière à être comprimée progressivement lorsqu'il est en appui sur la rampe du bord externe (7e) jusqu'à venir s'engager automatiquement dans la gorge d'arrêt (7a).

9. Système d'assemblage selon l'une des revendications précédentes, selon lequel le loquet (22) est destiné à s'engager automatiquement dans une gorge d'arrêt du bord externe (7e) d'une dent d'appui (7) sous l'effet de son propre poids.

10. Système d'assemblage selon l'une des revendications précédentes, selon lequel le loquet (22) est monté pour s'étendre verticalement à l'aplomb du milieu d'une dent de retenue (15) aménagée dans la plaque de blocage (10).

11. Matériel comportant un appareil (2), un support (3) et un système d'assemblage amovible (1) selon l'une des revendications précédentes, dans lequel l'appareil (2) est assemblé avec le support (3) à l'aide du système d'assemblage amovible (1), dans lequel la partie mâle (I) équipe le support (3) et la partie femelle (II) équipe l'appareil (2).

12. Matériel selon la revendication précédente, selon lequel l'appareil (2) comporte un boîtier (2₁) avec une face arrière pourvue de la partie femelle (II) du système d'assemblage dont la partie mâle (I) équipe le support (3).

13. Matériel selon la revendication précédente, selon lequel la face arrière du boîtier (2₁) de l'appareil est pourvue de l'organe (23) de déplacement en translation du loquet de verrouillage, positionné à proximité du bord supérieur du boîtier.

14. Matériel selon l'une des revendications 11 à 13, selon lequel l'appareil (2) est un écran de visualisation d'un endoscope médical.

## Patentansprüche

1. Lösbares Montagesystem (1) eines Geräts (2) mit einer Halterung (3) mittels eines Steckteils (I), der an der Halterung (3) vorhanden ist, und mit einem Buchsenteil (II) zusammenwirkt, der an dem Gerät (2) vorhanden ist;
- der Steckteil (I) umfassend einen Arm (5), der ein freies Ende (6) aufweist, das mit einer Reihe von Stützzähnen (7) versehen ist, die sich in einer radialen Ebene radial erstrecken und gemäß einer Winkelaufteilung verteilt sind;
- der Buchsenteil (II) umfassend mindestens eine Arretierplatte (10), die von einer Vorderseite (11) und einer Rückseite (12) begrenzt ist, und in der eine Öffnung (13) mit Querschnitt ausgebildet ist, der angepasst ist, um in einer Einführposition den Eingriff des freien Endes (6) des Steckteils von der Vorderseite aus über die Rückseite hinaus zuzulassen, wobei die in der Arretierplatte (10) ausgebildete Öffnung (13) Haltezähne (15) begrenzt, die sich radial erstrecken, und die nach einer relativen Drehung zwischen dem Buchsenteil (II) und dem Steckteil (I) in einer Arretierposition geeignet sind, um einem Stützzahn (7) gegenüber zu sein, um das Lösen zwischen dem Steck-und dem Buchsenteil von der Rückseite in Richtung der Vorderseite zu unterbinden;
- der Buchsenteil (II) und der Steckteil (I) umfassend eine Winkelverriegelungsvorrichtung (21), die geeignet ist, um den Buchsenteil (II) und den Steckteil (I) winkelmäßig zu verriegeln, wenn sie die Arretierposition einnehmen, die Winkelverriegelungsvorrichtung (21) umfassend eine Klinke (22), die radial beweglich an der Rückseite (12) der Arretierplatte (10) montiert und mit einem Element (23) zur translatorischen Verschiebung versehen ist, das von der Vorderseite (11) der Arretierplatte aus zugänglich ist, wobei der Klinke (22) dazu bestimmt ist, automatisch in eine Rastnut des äußeren Rands (7e) eines Stützzahns (7) einzugreifen;
- der Buchsenteil (II) umfassend einen Anschlagboden (18) für den Steckteil (I), der den Eingriff des Steckteils (I) über die Rückseite (12) der Arretierplatte (10) hinaus begrenzt und eine Drehführungsfläche zwischen dem Steckteil (I) und dem Buchsenteil (II) aufweist, um von der Einführposition in die Arretierposition und umgekehrt überzugehen.

2. Montagesystem nach dem vorherigen Anspruch, wobei der Anschlagboden (18) als Drehführungsfläche einen kreisförmigen, konisch geformten Ansatz umfasst.

3. Montagesystem nach einem der vorherigen Ansprüche, wonach der Steckteil (I) eine Anzahl von Stützzähnen (7) umfasst, die gleich ist wie die Anzahl von Haltezähnen (15) des Buchsenteils.

4. Montagesystem nach einem der vorherigen Ansprüche, wobei die Stützzähne (7) des Steckteils (I) und die Haltezähne (15) des Buchsenteils (II) winkelmäßig gleichförmig verteilt sind.

5. Montagesystem nach einem der vorherigen Ansprüche, wonach zwei benachbarte Haltezähne (15) des Buchsenteils (II) untereinander eine Durchgangskerbe (16) für einen Stützzahn (7) des Steckteils (I) begrenzen, während zwei benachbarte Stützzähne (7) des Steckteils (I) untereinander ein Loch (8) für den Durchgang eines Haltezahns (15) des Buchsenteils (II) begrenzen.

6. Montagesystem nach dem vorherigen Anspruch, wobei die Winkelbreite einer Durchgangskerbe (16), genommen zwischen zwei benachbarten Haltezähnen (15), gleich wie ein Wert ist, der zwischen 105 und 200 % der Winkelbreite liegt, die für einen Stützzahn (7) genommen wird.

7. Montagesystem nach dem vorherigen Anspruch, wobei der äußere Rand (7e) der Stützzähne (7) ausgehend von jedem seiner Enden eine Stützrampe für die Klinke (22) aufweist, die in eine Rastnut (7a) mündet.

8. Montagesystem nach Anspruch 1, wobei der Klinke (22) in seiner maximalen Erstreckungsposition durch ein Federelement elastisch zur Ausdehnung beansprucht wird, sodass sie allmählich zusammengedrückt wird, wenn sie auf der Rampe des äußeren Rands (7e) aufliegt, bis sie automatisch in die Rastnut (7a) eingreift.

9. Montagesystem nach einem der vorherigen Ansprüche, wobei die Klinke (22) dazu bestimmt ist, unter der Wirkung seines Eigengewichts automatisch in eine Rastnut des äußeren Rands (7e) eines Stützzahns (7) einzugreifen.

10. Montagesystem nach einem der vorherigen Ansprüche, wobei die Klinke (22) montiert ist, um sich vertikal lotrecht zu der Mitte eines in der Arretierplatte (10) ausgebildeten Haltezahns (15) zu erstrecken.

11. Material, umfassend ein Gerät (2), eine Halterung (3) und ein lösbares Montagesystem (1) nach einem der vorherigen Ansprüche, wobei das Gerät (2) mittels des abnehmbaren Montagesystems (1) mit der Halterung (3) zusammengebaut wird, wobei der Steckteil (I) an der Halterung (3) vorhanden ist und der Buchsenteil (II) an dem Gerät (2) vorhanden ist.

12. Material nach dem vorherigen Anspruch, wobei das Gerät (2) ein Gehäuse (2₁) mit einer Rückseite umfasst, die mit dem Buchsenteil (II) des Montagesystems versehen ist, dessen Steckteil (I) an der Halterung (3) vorhanden ist.

13. Material nach dem vorherigen Anspruch, wobei die Rückseite des Gehäuses (2₁) des Geräts mit dem Element (23) zur translatorischen Bewegung der Sperrklinke versehen ist, das in der Nähe des oberen Rands des Gehäuses positioniert ist.

14. Material nach einem der Ansprüche 11 bis 13, wobei das Gerät (2) ein Anzeigebildschirm eines medizinischen Endoskops ist.

## Claims

1. A removable system (1) for joining a device (2) to a support (3), using a male part (I) equipping the support (3) and interacting with a female part (II) equipping the device (2);
- the male part (I) including an arm (5) having a free end (6) provided with a series of bearing teeth (7) extending radially in a radial plane and distributed over an angular distribution;
- the female part (II) including at least one locking plate (10) delimited by a front face (11) and a back face (12) and in which is arranged an opening (13) of a section suitable for allowing, in an insertion position, the engagement, from the front face, of the free end (6) of the male part beyond the back face, the opening (13) arranged in the locking plate (10) delimiting retaining teeth (15) extending radially and which, after a relative rotation between the female part (II) and the male part (I), are able, in a locking position, to come face-to-face with a bearing tooth (7) to prevent the disengagement, between the male and female parts, of the back face in the direction of the front face,
- the female part (II) and the male part (I) including an angular locking device (21) able to angularly lock the female (II) and male (I) parts when they are occupying the locking position, the angular locking device (21) including a latch (22) mounted radially movable on the back face (12) of the locking plate (10) and provided with a translating member (23) accessible from the front face (11) of the locking plate, the latch (22) being intended to automatically engage in a limiting groove of the external edge (7e) of a bearing tooth (7);
- the female part (II) including an abutment bottom (18) for the male part (I) limiting the engagement of the male part (I) beyond the back face (12) of the locking plate (10) and having a rotational guiding surface between the male part (I) and the female part (II) in order to change from the insertion position to the locking position and conversely.

2. The joining system as claimed in the preceding claim, wherein the abutment bottom (18) includes, as a rotational guiding surface, a circular stud of conical shape.

3. The joining system as claimed in one of the preceding claims, wherein the male part (I) includes a number of bearing teeth (7) equal to the number of retaining teeth (15) of the female part.

4. The joining system as claimed in one of the preceding claims, wherein the bearing teeth (7) of the male part (I) and the retaining teeth (15) of the female part (II) are regularly angularly distributed.

5. The joining system as claimed in one of the preceding claims, wherein two neighboring retaining teeth (15) of the female part (II) together delimit a notch (16) for passing a bearing tooth (7) of the male part (I) while two neighboring bearing teeth (7) of the male part (I) together delimit a hole (8) for passing a retaining tooth (15) of the female part (II).

6. The joining system as claimed in the preceding claim, wherein the angular width of a passing notch (16) measured between two neighboring retaining teeth (15) is equal to a value between 105 to 200% of the angular width measured for a bearing tooth (7).

7. The joining system as claimed in the preceding claim, wherein the external edge (7e) of the bearing teeth (7) includes from each of its ends a bearing rail for the latch (22) opening into a limiting groove (7a).

8. The joining system as claimed in claim 1, wherein the latch (22) is elastically stressed in extension by a spring member in its maximum extension position in such a way as to be gradually compressed when it is bearing against the rail of the external edge (7e) until it comes to automatically engage in the limiting groove (7a).

9. The joining system as claimed in one of the preceding claims, wherein the latch (22) is intended to automatically engage in a limiting groove of the external edge (7e) of a bearing tooth (7) under the effect of its own weight.

10. The joining system as claimed in one of the preceding claims, wherein the latch (22) is mounted to extend vertically plumb with the middle of a retaining tooth (15) arranged in the locking plate (10).

11. Equipment including a device (2), a support (3), and a removable joining system (1) according to one of the preceding claims, wherein the device (2) is assembled with the support (3) using the removable joining system (1), wherein the male part (I) equips the support (3) and the female part (II) equips the device (2).

12. Equipment as claimed in the preceding claim, wherein the device (2) includes a casing (2₁) with a back face provided with the female part (II) of the joining system, the male part (I) of which equips the support (3).

13. Equipment as claimed in the preceding claim, wherein the back face of the casing (2₁) of the device is provided with the translating member (23) of the locking latch, positioned near the upper edge of the casing.

14. Equipment as claimed in one of claims 11 to 13, wherein the device (2) is a display screen of a medical endoscope.
